# EUROPÄISCHE PATENTSCHRIFT

(11) **EP 2 403 363 B1**
(45) Veröffentlichungstag und Bekanntmachung des Hinweises auf die Patenterteilung: **26.12.2012**
(21) Anmeldenummer: 10707514.5
(22) Anmeldetag: 04.03.2010
(51) Int. Cl.: A23L 1/275, C07C 403/24, A23K 1/16

(54) **FORMULIERUNG VON ASTAXANTHIN-DERIVATEN UND DEREN VERWENDUNG II**
FORMULATION OF ASTAXANTHIN DERIVATIVES AND USE THEREOF II
FORMULATION DE DÉRIVÉS D'ASTAXANTHINE ET LEUR UTILISATION II

(30) Priorität: 05.03.2009 EP 09154435
(43) Veröffentlichungstag der Anmeldung: 11.01.2012
(73) Patentinhaber: BASF SE, 67056 Ludwigshafen (DE)
(72) Erfinder: FELDTHUSEN JENSEN, Jesper, 55268 Nieder-Olm (DE); END, Lutz, 68526 Ladenburg (DE); KÖPSEL, Christian, 69469 Weinheim (DE); BRANDS, MARIO, 67063 Ludwigshafen (DE); ENGEL, Robert, 67346 Speyer (DE); GOTTSCHALK, Thomas, 68165 Mannheim (DE); PELLETIER, Wolf, 76879 Ottersheim (DE)
(86) Internationale Anmeldenummer: PCT/EP2010/052757
(87) Internationale Veröffentlichungsnummer: WO 2010/100227

(56) Entgegenhaltungen:
- WO-A1-03/066583
- WO-A1-2006/125591
- GB-A- 803 077

## Beschreibung

Die vorliegende Erfindung betrifft feste Formulierungen von Astaxanthin-Derivaten sowie die Verwendung der Formulierungen zur Herstellung von Lösungen des Astaxanthin-Derivats in für Nahrungsmittel geeigneten Ölen sowie zur Herstellung von Futtermitteln. Die Erfindung betrifft auch ein Verfahren zur Herstellung derartiger Formulierungen und ein Verfahren zur Herstellung von Futtermitteln unter Verwendung der festen Formulierungen.

Astaxanthin (3,3'-Dihydroxy-β,β-carotin-4,4'-dion) und seine Derivate, insbesondere seine Ester, sind als Nahrungs- und Futtermittelzusatzstoffe von Interesse. So wird Astaxanthin in großem Umfang bei der Aufzucht von Speisefischen in Aquakulturen (Fischfarmen) als Futtermittelzusatz eingesetzt. Aufgrund der vitaminartigen Eigenschaft von Astaxanthin und seinen Derivaten wirken sie sich vorteilhaft auf die Gesundheit der Fische in den Aquakulturen aus und fördern deren Fruchtbarkeit. Zudem bewirken Astaxanthin und seine Derivate eine Intensivierung der Färbung von Fleisch und Haut der Fische, was nicht zuletzt aus ästhetischen und kulinarischen Gründen wünschenswert ist.

Problematisch sind die geringe Wasserlöslichkeit von Astaxanthin und seinen Derivaten und ihre damit einhergehende schlechte Bioverfügbarkeit. Aus diesem Grund können Astaxanthin und seine Derivate nicht als solche eingesetzt werden, sondern müssen in eine Formulierung überführt werden, die eine hinreichende Bioverfügbarkeit dieser Substanzen gewährleistet. Aufgrund der chemischen Instabilität vom Astaxanthin und seinen Derivaten stellt jedoch die Formulierung dieser Verbindungen eine besondere Herausforderung dar.

Für verschiedene Anwendungszwecke, insbesondere zur Futtermittelherstellung, hat es sich grundsätzlich als vorteilhaft erwiesen, Astaxanthin oder seine Derivate in Form von verdünnten Lösungen in für Nahrungsmittel geeigneten, flüssigen Ölen bereitzustellen. Diese verdünnten Lösungen können dann bei der Futtermittelherstellung verwendet werden und gewährleisten eine hinreichende Bioverfügbarkeit in dem Futtermittel.

Bei der Herstellung derartiger Lösungen erweist sich die schlechte Lösungskinetik des Astaxanthins als problematisch.

Die WO 03/102116 beschreibt ölige Lösungen von Carotinoiden wie Astaxanthin. Ihre Herstellung erfolgt durch Lösen des Carotinoids in einem organischen Lösungsmittel wie N-Methylpyrrolidon in Anwesenheit eines lipophilen Dispergiermittels und Entfernen des Lösungsmittels. Das erhaltene Pulver wird dann in einem Öl, beispielsweise Fischöl, gelöst. Dieses Verfahren ist vergleichsweise aufwändig und bedarf größerer Mengen an Schutzkolloiden.

Die WO 2006/125591 beschreibt die Herstellung von öligen Carotinoid-Lösungen, beispielsweise Lösungen des Astaxanthins, bei dem man zunächst eine Suspension des Carotinoids in der Ölphase bereitstellt, die Suspension für eine kurze Zeit auf hohe Temperaturen, z. B. im Bereich von 100 bis 230 °C erhitzt, um das Carotinoid in Lösung zu bringen, und die heiße Lösung mit einem kalten Öl vermischt. Zur Herstellung von öligen Lösungen des Astaxanthins werden regelmäßig Temperaturen oberhalb 160 °C angewendet. Dieses Verfahren ist mit einer Reihe von Nachteilen verbunden. Zum einen führt das Erhitzen aufgrund der chemischen Labilität von Carotinoiden unter anderem zu einer unerwünschten cis-trans-Isomerisierung der exocyclischen Doppelbindungen und damit zu einem Aktivitätsverlust. Zudem erweist sich die Bereitstellung öliger Suspensionen des Astaxanthins durch Vermahlen von Astaxanthin in Öl als problematisch.

Der vorliegenden Erfindung liegt die Aufgabe zu Grunde, geeignete Formulierungen von Astaxanthin oder einem Astaxanthin-Derivat bereitzustellen, die es erlauben, Astaxanthin oder das Astaxanthin-Derivat in ein für Nahrungsmittel geeignetes Öl einzuarbeiten.

Die ältere internationale Patentanmeldung PCT/EP2008/061384 beschreibt ein Verfahren zur Herstellung von öligen Lösungen des Astaxanthins in für Nahrungsmittel geeigneten Ölen, bei denen man bestimmte Astaxanthin-Derivate der im Folgenden angegebenen Formel I zunächst in eine Suspension in einem essbaren Öl überführt, wobei die Partikel des Astaxanthin-Derivats in der Suspension einen volumenmittleren Teilchendurchmesser, bestimmt durch Fraunhofer-Beugung, im Bereich von 0,5 bis 50 µm aufweisen. Diese Suspensionen sind typischerweise bei Temperaturen von 25 °C flüssig und können in einfacher Weise mit flüssigen, für Nahrungsmittel geeigneten Ölen verdünnt werden, wobei sich die verwendeten Astaxanthin-Derivate aufgrund ihrer vorteilhaften Lösungskinetik und der geringen Partikelgröße rasch in dem zur Verdünnung eingesetzten Öl auflösen, ohne dass es der Anwendung von Temperaturen oberhalb 100 °C und/oder größerer Mengen an Emulgatoren bedarf.

Es wurde überraschenderweise gefunden, dass die im Folgenden definierten Astaxanthin-Derivate der Formel I, sowie Astaxanthin-Derivate, welche zu wenigstens 70 Gew.-% aus wenigstens einer Verbindung der Formel I bestehen, die aus PCT/EP2008/061384 bekannten Vorteile auch dann aufweisen, wenn man sie in Form einer festen Suspension oder Lösung des Astaxanthin-Derivats in einem für Nahrungsmittel geeigneten Verdünnungsmittel, das einen Schmelzpunkt von wenigstens 40 °C, vorzugsweise wenigstens 50 °C, insbesondere wenigstens 55 °C aufweist und das unter für Nahrungsmittel geeigneten Fettsäuren, Fettsäureestern, Fettalkoholen und Wachsen mit einem Schmelzpunkt von wenigstens 40 °C, vorzugsweise wenigstens 50 °C, insbesondere wenigstens 55 °C und deren Gemischen ausgewählt ist, bereitstellt. Bei den Astaxanthin-Derivaten handelt es sich um Verbindungen der folgenden Formel I 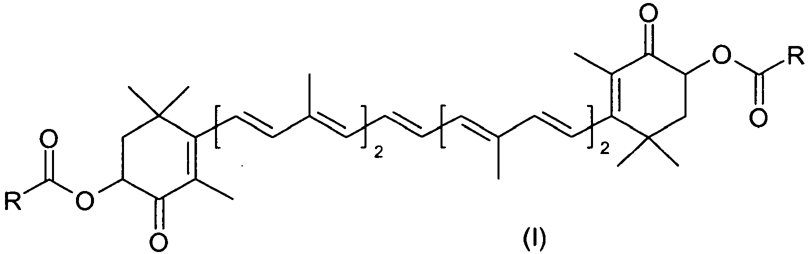 worin R für einen Rest der Formel 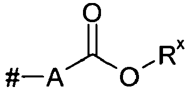 steht, worin # die Anknüpfung an die Carbonylgruppe bedeutet, A für -CH₂CH₂- oder für -CH=CH- steht und R^{x} für C₁-C₄ Alkyl steht; sowie um Astaxanthin-Derivate, die zu wenigstens 70 Gew.-% aus den Verbindungen der Formel I bestehen.

Derartige Astaxanthin-Derivate und Verfahren zu ihrer Herstellung sind aus der WO 03/066583 A1 bekannt.

Demnach betrifft die vorliegende Erfindung feste Formulierungen von Astaxanthin-Derivaten in Form einer festen Suspension oder einer festen Lösung des Astaxanthin-Derivats in einem für Nahrungsmittel geeigneten Verdünnungsmittel, das einen Schmelzpunkt von wenigstens 40 °C, vorzugsweise wenigstens 50 °C, insbesondere wenigstens 55 °C aufweist und das unter für Nahrungsmittel geeigneten Fettsäuren, Fettsäureestern, Fettalkoholen und Wachsen mit einem Schmelzpunkt von wenigstens 40 °C, vorzugsweise wenigstens 50 °C, insbesondere wenigstens 55 °C und deren Gemischen ausgewählt ist, wobei das Astaxanthin-Derivat zu wenigstens 70 Gew.-%, bevorzugt zu wenigstens 80 Gew.-%, insbesondere zu wenigstens 90 Gew.-% und speziell zu wenigstens 96 Gew.-% aus wenigstens einer Verbindung der Formel I besteht.

Die Erfindung ist mit einer Reihe von Vorteilen verbunden. Zum einen lassen sich unter Verwendung der erfindungsgemäßen festen Formulierungen in einfacher Weise Lösungen von Astaxanthin-Derivaten in flüssigen, für Nahrungsmittel geeigneten Ölen durch Verdünnen (bzw. Einarbeiten) der erfindungsgemäßen festen Formulierungen mit dem (in das) für Nahrungsmittel geeignete flüssige Öl herstellen. Anders als in dem in WO 2006/125591 beschriebenen Verfahren bedarf es hierzu grundsätzlich nicht der Anwendung von Temperaturen oberhalb 100 °C. Vielmehr kann man unter Verwendung der erfindungsgemäßen Formulierungen verdünnte Lösungen des Astaxanthin-Derivats in dem flüssigen Öl bei Temperaturen von unterhalb 100 °C, insbesondere bei maximal 90 °C oder gar maximal 80 °C, z. B. bei Temperaturen im Bereich von 40 bis 90 °C, insbesondere 60 bis 85 °C herstellen. Aufgrund der niedrigen Temperaturen zeichnen sich die auf diese Weise erhältlichen Lösungen durch einen sehr hohen Anteil an all-trans-Isomeren aus, der in der Regel oberhalb 80 %, insbesondere oberhalb 90 %, bezogen auf das in der Formulierung enthaltene Astaxanthin-Derivat liegt. Ferner lassen sich unter Verwendung von Astaxanthin-Derivaten, die zu wenigstens 70 Gew.-% aus wenigstens einer Verbindungen der Formel I bestehen, sehr viel einfacher feste Formulierungen von Astaxanthin-Derivaten herstellen als bei Verwendung von Astaxanthin selber oder von anderen, davon verschiedenen Astaxanthin-Derivaten wie Astaxanthindiacetat, Astaxanthindisuccinat oder Astaxanthindipalmitat.

Die erfindungsgemäßen festen Formulierungen haben neben einer guten Verdünnbarkeit in den für Nahrungsmittel geeigneten Ölen den Vorteil einer verbesserten Handhabbarkeit und Stabilität im Vergleich zu den aus PCT/EP2008/061384 bekannten flüssigen Suspensionen.

In Formel I steht C₁-C₄-Alkyl für einen linearen oder verzweigten, gesättigten Kohlenwasserstoffrest mit 1 bis 4 C-Atomen wie Methyl, Ethyl, n-Propyl, 1-Methylethyl, Butyl, 1-Methylpropyl, 2-Methylpropyl oder 1,1-Dimethylethyl.

Die beiden Reste R in Formel I können gleich oder verschieden sein und sind vorzugsweise identisch.

Vorzugsweise steht A für 1,2-Ethandiyl. R^{x} steht vorzugsweise für Methyl oder Ethyl.

Verbindungen der Formel I, worin A im Rest R für 1,2-Ethandiyl steht, werden im Folgenden auch als Dialkyldisuccinate bezeichnet. Hierunter besonders bevorzugt ist das Dimethylsuccinat.

Die Verbindungen der Formel I können, da die die Gruppe O-C(O)R tragenden Kohlenstoffatome Asymmetriezentren sind, diastereomere und enantiomere Formen bilden, nämlich die 3R,3'S-Form, die 3S,3'R-Form, die 3R,3'R-Form und die 3S,3'S-Form. Sofern die Reste R in Formel I gleich sind, sind die 3R,3'S-Form und die 3S,3R-Form identisch und achiral (meso-Form) und die 3S,3'S-Form und die 3R,3'R-Form bilden ein Enantiomerenpaar. Für die Erfindung können sowohl die reinen Enantiomere und Diastereomere sowie Gemische der vorgenannten Diastereomere, z. B. ein racemisches Gemisch der 3S,3'S-Form und der 3R,3'R-Form als auch ein Gemisch der meso-Form mit der 3S,3'S-Form und/oder der 3R,3'R-Form oder ein Gemisch der meso-Form und dem Racemat der 3S,3'S-Form und der 3R,3'R-Form eingesetzt werden. In einer bevorzugten Ausgestaltung der Erfindung wird die Verbindung der Formel I in der 3S,3'S-Form oder einer Mischung der 3S,3'S-Form mit einer oder weiteren Formen der Verbindung der Formel I eingesetzte, worin die 3S,3'S-Form wenigstens 80 Gew.-%, insbesondere wenigstens 90 Gew.-%, bezogen auf die Gesamtmenge der Verbindung der Formel I ausmacht. In einer weiteren bevorzugten Ausgestaltung der Erfindung wird die Verbindung der Formel I in der 3R,3'R-Form oder einer Mischung der 3R,3'R-Form mit einer oder weiteren Formen der Verbindung der Formel I eingesetzt, worin die 3R,3'R-Form wenigstens 80 Gew.-%, insbesondere wenigstens 90 Gew.-%, bezogen auf die Gesamtmenge der Verbindung der Formel I ausmacht.

Die erfindungsgemäßen Formulierungen enthalten in der Regel 0,5 bis 50 Gew.-%, insbesondere 1 bis 30 Gew.-% und speziell 1,5 bis 25 Gew.-%, bezogen auf das Gesamtgewicht der Formulierung, wenigstens eines Astaxanthin-Derivats, das zu wenigstens 70 Gew.-%, häufig zu wenigstens 80 Gew.-%, vorzugsweise zu wenigstens 90 Gew.-%, insbesondere zu wenigstens 96 Gew.-%, bezogen auf die Gesamtmenge an Astaxanthin-Derivat in der Zubereitung, aus einer Astaxanthin-Verbindung der Formel I besteht. Im Falle der Suspensionen enthalten die erfindungsgemäßen Formulierungen vorzugsweise 1 bis 50 Gew.-%, insbesondere 2 bis 30 Gew.-% und speziell 3 bis 25 Gew.-%, bezogen auf das Gesamtgewicht der Formulierung, wenigstens eines feinteiligen Astaxanthin-Derivats. Im Falle der Lösungen wird die Konzentration des Astaxanthin-Derivats in der Regel 5 Gew.-% und insbesondere 4 Gew.-% nicht überschreiten und liegt typischerweise im Bereich von 0,5 bis 5 Gew.-%, insbesondere bei 1 bis 4 Gew.-%.

In den erfindungsgemäßen Formulierungen weist das Astaxanthin-Derivat üblicherweise eine Reinheit, bezogen auf das in Formel I gezeigten all-trans-Isomer, von wenigstens 70 Gew.-%, häufig wenigstens 80 Gew.-%, vorzugsweise wenigstens 90 Gew.-% auf. Neben dem all-trans-Isomeren kann das Astaxanthin-Derivat auch Anteile an Astaxanthin-Verbindungen der Formel I enthalten, in denen eine oder mehrere Doppelbindungen der in Formel I dargestellten all-trans-Isomere cis-Konfiguration aufweisen. Die Gesamtmenge an all-trans-Isomer der Formel I und cis-Isomeren der Formel I beträgt in der Regel wenigstens 80 Gew.-%, häufig wenigstens 90 Gew.-%, insbesondere wenigstens 96 Gew.-%. Neben dem all-trans-Isomeren der Formel I und etwaigem cis-Isomeren kann das Astaxanthin-Derivat auch weitere Carotinoide umfassen, wobei der Anteil dieser Verunreinigungen in der Regel 30 Gew.-%, häufig 20 Gew.-%, insbesondere 10 Gew.-%, besonders bevorzugt 4 Gew.-%, bezogen auf die Gesamtmenge des eingesetzten Astaxanthin-Derivats nicht überschreitet. Bei diesen Verunreinigungen handelt es sich in erster Linie um Halbester des Astaxanthins, d. h. um Verbindungen der im Folgenden gezeigten Formel II 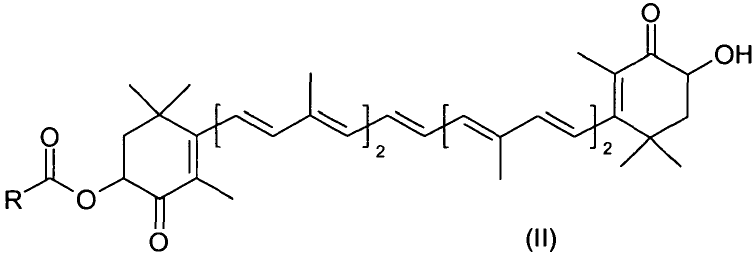 worin R die zuvor genannten Bedeutungen hat bzw. um cis-Isomere davon, sowie um Astaxanthin selber, Adonirubin und/oder Semi-Astacin.

Insbesondere enthält die erfindungsgemäße Formulierung ein Astaxanthin-Derivat, das die an ein für Nahrungsmittel, insbesondere für Futtermittel zugelassenes Astaxanthin gestellten Anforderungen erfüllt, d. h. das weniger als 4 Gew.-% Carotinoide, die von Astaxanthin und seinen Derivaten verschieden sind, enthält und das einen Schwermetallgehalt von höchstens 10 ppm aufweist, das zu wenigstens 70 Gew.-%, häufig zu wenigstens 80 Gew.-%, vorzugsweise zu wenigstens 90 Gew.-%, insbesondere zu wenigstens 96 Gew.-%, bezogen auf die Gesamtmenge an Astaxanthin-Derivat in der Zubereitung, aus einer Astaxanthin-Verbindung der Formel I besteht, wobei das in der Zusammensetzung enthaltene Astaxanthin-Derivat zu wenigstens 70 Gew.-%, häufig wenigstens 80 Gew.-% und vorzugsweise wenigstens 90 Gew.-% aus dem all-trans-Isomeren besteht.

Bei den erfindungsgemäßen Formulierungen handelt es sich um feste Suspensionen oder Lösungen des Astaxanthin-Derivats in einem Verdünnungsmittel, das einen Schmelzpunkt von wenigstens 40 °C, vorzugsweise wenigstens 50 °C, insbesondere wenigstens 55 °C, z.B. im Bereich von 40 °C bis 100 °C, vorzugsweise im Bereich von 50 °C bis 90 °C, insbesondere im Bereich von 55 °C bis 85 °C aufweist.

Bei dem Verdünnungsmittel handelt es sich um für Nahrungsmittel zugelassene Substanzen auf Basis von Fettsäuren, Fettsäureestern, Fettalkoholen, Wachsen und Gemischen davon. Hierbei handelt es sich um feste organische Substanzen, die langkettige ungesättigte und/oder vorzugsweise gesättigte Kohlenwasserstoffketten mit in der Regel wenigstens 11 C-Atomen, z. B. 11 bis 30 C-Atomen, insbesondere 13 bis 28 C-Atomen, aufweisen, die mit Wasser nicht oder nur begrenzt mischbar sind, und die üblicherweise unzersetzt bei Temperaturen von in der Regel maximal 100 °C schmelzen. Die Verbindungen sind naturgemäß aus Kohlenstoff, Wasserstoff, Sauerstoff und gegebenenfalls Stickstoff aufgebaut und enthalten darüber hinaus keine weiteren Elemente. Der Anteil an gesättigten Kohlenwasserstoffketten in den liegt in der Regel bei wenigstens 60 Gew.-%, insbesondere wenigstens 70 Gew.-% und speziell wenigstens 80 Gew.-%, bezogen auf das Gesamtgewicht des Verdünnungsmittels. Der Anteil an ungesättigten Kohlenwasserstoffketten in den liegt in der Regel bei maximal 20 Gew.-%, insbesondere maximal 10 Gew.-% und speziell maximal 1 Gew.-%, bezogen auf das Gesamtgewicht des Verdünnungsmittels.

Der Begriff "fest" im Hinblick auf die erfindungsgemäßen Formulierungen und Verdünnungsmittel bedeutet, dass sie sich bei Normalbedingungen (298,15 K, 1013 hPa) im festen Aggregatzustand befinden.

Unter einer für Nahrungsmittel zugelassenen Substanz versteht man im Sinne der Erfindung eine für die Tier- und/oder Humanernährung zugelassene Substanz. Der Begriff "Nahrungsmittel" umfasst hier und im Folgenden Nahrungsmittel für die Humanernährung (= Lebensmittel) und Nahrungsmittel für die Tierernährung (= Futtermittel).

Geeignete Fettsäuren als Bestandteil des Verdünnungsmittels der erfindungsgemäßen Formulierungen sind solche, die einen Schmelzpunkt von wenigsten 40 °C, vorzugsweise wenigstens 50 °C, insbesondere wenigstens 55 °C, z.B. im Bereich von 40 °C bis 100 °C, vorzugsweise im Bereich von 50 °C bis 90 °C, insbesondere im Bereich von 55 °C bis 80 °C aufweisen. Vorzugsweise handelt es sich um gesättigte Fettsäuren mit 12 bis 30 C-Atomen (gesättigte C₁₂-C₃₀-Fettsäuren), insbesondere 14 bis 28 C-Atomen (gesättigte C₁₄-C₂₈-Fettsäuren) und speziell 16 bis 24 C-Atomen (gesättigte C₁₆-C₂₄-Fettsäuren) wie Myristinsäure, Palmitinsäure, Margarinsäure, Stearinsäure, Arachinsäure, Behensäure, Cerotinsäure, Melissinsäure und Lignocerinsäure sowie deren Gemische.

Geeignete Fettsäureester als Bestandteil des Verdünnungsmittels der erfindungsgemäßen Formulierungen sind solche, die einen Schmelzpunkt von wenigstens 40 °C, vorzugsweise wenigstens 50 °C, insbesondere wenigstens 55 °C, z. B. im Bereich von 40 °C bis 100 °C, vorzugsweise im Bereich von 50 °C bis 90 °C, insbesondere im Bereich von 55 bis 85 °C aufweisen. Vorzugsweise handelt es sich um Ester von gesättigten Fettsäuren mit 14 bis 30 C-Atomen, insbesondere 14 bis 28 C-Atomen und speziell 16 bis 24 C-Atomen wie die Ester der Palmitinsäure, Margarinsäure, Stearinsäure, Arachinsäure, Behensäure, Cerotinsäure, Melissinsäure und Lignocerinsäure sowie deren Gemische.

Zu den Estern der Fettsäuren zählen insbesondere die Ester des Glycerins mit einer oder mehrerer der vorgenannten Fettsäuren, d. h. Mono-, Di- und Triglyceride der vorgenannten Fettsäuren (im Folgenden als Fettsäure-Glyceride bezeichnet), insbesondere der vorgenannten gesättigten Fettsäuren und speziell solcher gesättigter Fettsäuren mit 14 bis 28 C-Atomen und speziell 16 bis 28 C-Atomen und Ester von Fettsäuren, insbesondere der vorgenannten gesättigten Fettsäuren und speziell solcher gesättigter Fettsäuren mit 14 bis 28 C-Atomen und speziell 16 bis 28 C-Atomen, mit Fettalkoholen, die vorzugsweise 10 bis 30 C-Atome (C₁₀-C₃₀-Fettalkohole), insbesondere 14 bis 28 C-Atome (C₁₄-C₂₈-Fettalkohole) und speziell 16 bis 26 C-Atome (C₁₆-C₂₆-Fettalkohole) aufweisen.

Die vorgenannten Ester von gesättigten Fettsäuren können bis zu 10 Gew.-%, bezogen auf die Fettsäureanteil im Ester, auch ein oder mehrfach ungesättigte Fettsäuren in veresterter Form enthalten. Insbesondere macht der Anteil ungesättigter Fettsäurebestandteile in diesen Estern weniger als 5 Gew.-% aus, bezogen auf die gesamten Fettsäurebestandteile im Ester.

Als Verdünnungsmittel sind weiterhin Fettalkohole, insbesondere gesättigte Fettalkohole geeignet, die vorzugsweise 16 bis 30 C-Atome aufweisen (C₁₆-C₃₀-Fettalkohole), wie beispielsweise Cetylalkohol, Stearylalkohol, Nonadecanol, Arachidylalkohol, Behenylalkohol, Lignocerylalkohol, Cerylalkohol, Myricylalkohol und Melissylalkohol.

Als Verdünnungsmittel sind weiterhin Wachse, insbesondere soweit nicht bereits in den vorgenannten Gruppen enthalten, geeignet. Hierzu zählen insbesondere natürliche Wachse pflanzlichen oder tierischen Ursprungs wie Bienenwachs, Candelillawachs, Schellack-Wachs, Karitefett und Carnaubawachs, Kohlenwasserstoffwachse, wie Paraffinwachse, Ceresin, Sasolwachse, Ozokerit und Mikrowachse.

Als Verdünnungsmittel sind auch Gemische der vorgenanten Substanzen geeignet, solange das Gemisch einen Schmelzpunkt von wenigstens 40 °C, vorzugsweise wenigstens 50 °C, insbesondere wenigstens 55 °C, z.B. im Bereich von 40 °C bis 100 °C, vorzugsweise im Bereich von 50 °C bis 90 °C, insbesondere im Bereich von 55 °C bis 85 °C aufweist.

Als Verdünnungsmittel sind Fettsäureester des Glycerins mit wenigstens einer der vorgenannten Fettsäuren, insbesondere der vorgenannten gesättigten Fettsäuren und speziell solcher gesättigter Fettsäuren mit 14 bis 28 C-Atomen und speziell 16 bis 24 C-Atomen sowie deren Gemische bevorzugt, wobei der Anteil gesättigter Fettsäuren, bezogen auf die Gesamtmenge der Fettsäuren im Ester, vorzugsweise wenigstens 90 Gew.-% und insbesondere wenigstens 95 Gew.-% beträgt. Bevorzugte Fettsäure-Glyceride weisen eine Säurezahl (bestimmt nach Ph. Eur.) von < 1 mg KOH/g auf. Besonders bevorzugt handelt es sich bei dem Verdünnungsmittel um ein Hartfett oder ein Gemisch von Hartfetten, insbesondere um ein Hartfett oder ein Gemisch von Hartfetten mit einer Säurezahl (bestimmt nach Ph. Eur.) von < 0,5 mg KOH/g. Vorzugsweise weisen die Fettsäure-Glyceride eine Jodzahl von maximal 10, insbesondere maximal 5 auf. Bevorzugte Fettsäure-Glyceride weisen eine Peroxidzahl (bestimmt nach Ph. Eur.) von maximal 10 meq. O₂/kg, von maximal 5 meq. O₂/kg und speziell von maximal 1 meq. O₂/kg auf. Beispiele für derartige Fettsäure-Glyceride sind gehärtetes Talgfett, gehärteter Rindertalg, Gemische aus Mono- und Diglyceriden der Palmitin- und der Stearinsäure, hydriertes Karitéfett, hydrierte C₁₄-C₁₈-Triglycerid-Gemische und hydriertes Rapsöl.

Als Verdünnungsmittel sind weiterhin Ester der vorgenannten Fettsäuren mit Fettalkoholen (im Folgenden auch als Fettalkohol-Ester bezeichnet), insbesondere Ester der vorgenannten gesättigten Fettsäuren und speziell solcher gesättigter Fettsäuren mit 14 bis 28 C-Atomen und speziell 16 bis 28 C-Atomen mit gesättigten Fettalkoholen mit 10 bis 28 C-Atomen und speziell 12 bis 28 C-Atomen sowie deren Gemische und deren Gemische mit den vorgenannten Fettsäureestern des Glycerins bevorzugt, wobei der Anteil gesättigter Fettsäuren, bezogen auf die Gesamtmenge der Fettsäuren im Ester, vorzugsweise wenigstens 90 Gew.-% und insbesondere wenigstens 95 Gew.-% beträgt. Bevorzugte Fettsäureester von Fettalkholen sind Cetylstearat und Cetylpalmitat. Geignet sind insbesondere auch Gemische aus Fettalkohol-Estern mit Fettsäuren und/oder mit Fettsäureglyceriden.

Gemäß einer Ausführungsform der Erfindung umfasst das Verdünnungsmittel kein oder keine nennenswerten Mengen (< 10 Gew.-%, insbesondere < 1 Gew.-%, bezogen auf die Gesamtmenge an Verdünnungsmittel) an Presstalg (Oleostearin) und kein oder keine nennenswerten Mengen (< 10 Gew.-%, insbesondere < 1 Gew.-%, bezogen auf die Gesamtmenge an Verdünnungsmittel) an Glycerylstearat.

Der Anteil an Verdünnungsmittel am Gesamtgewicht der Formulierung liegt typischerweise im Bereich von 50 bis 99,5 Gew.-%, vorzugsweise im Bereich von 70 bis 99 Gew.-% und insbesondere im Bereich von 75 bis 98,5 Gew.-%.

Neben dem Astaxanthin-Derivat können die erfindungsgemäßen Formulierungen auch weitere Bestandteile enthalten, wie sie für diese Zwecke üblicherweise geeignet sind und angewendet werden. Hierzu zählen lipophile Dispergiermittel, Antioxidantien (Oxidationsstabilisatoren) und dergleichen erfolgen. Beispiele für Antioxidantien sind Tocopherole wie α-Tocopherol, α-Tocopherolpalmitat, α-Tocopherolacetat, tert.-Butylhydroxytoluol, tert.-Butylhydroxyanisol, Ascorbinsäure, deren Salze und Ester wie z. B. Natriumascorbat, Calciumascorbat, Ascorbylphosphatester und Ascorbylpalmitat und Ethoxyquin. Die Antioxidantien sind, sofern erwünscht, in der erfindungsgemäßen Formulierung typischerweise in Mengen von 0,01 bis 10 Gew.-%, bezogen auf das Gesamtgewicht der Formulierung, enthalten. Typische lipophile Dispergiermittel sind Ascorbylpalmitat, Polyglycerin-Fettsäureester wie Polyglycerin-3-polyrizinoleat (PGPR90), Sorbitanfettsäureester, insbesondere Sorbitan-C₁₀-C₂₈-fettsäureester wie z.B. Mono- und Di-C₁₀-C₂₈-fettsäureester des Sorbitans wie Sorbitanmonolaurat, Sorbitanmonooleat und Sorbitanmonostearat (SPAN60), ethoxilierte Sorbitanfettsäureester wie PEG(20)-Sorbitolmonooleat, Monoester der Milchsäure mit gesättigten C₁₀-C₂₄-Fettsäuren, Zuckerester von gesättigten C₁₆-C₂₈-Fettsäuren, Propylenglycol-Fettsäureester sowie Phospholipide wie Lecithin. Lipophile Dispergiermittel sind, sofern erwünscht, in der erfindungsgemäßen Formulierung typischerweise in Mengen von 0,01 bis 10 Gew.-%, bezogen auf das Gesamtgewicht der Formulierung, enthalten. Lipophile Dispergiermittel werden, sofern erwünscht, üblicherweise in Mengen von 0,1 bis 10 Gew.-Teilen, bezogen auf 1 Gew.-Teil des Astaxanthin-Derivats, eingesetzt. In einer bevorzugten Ausführungsform der Erfindung enthält die Formulierung im Wesentlichen kein, d. h. weniger als 0,5 Gew.-%, bezogen auf die Formulierung, lipophiles Dispergiermittel.

In den erfindungsgemäßen Formulierungen kann das Astaxanthin-Derivat in dem festen Verdünnungsmittel in gelöster und/oder suspendierter Form vorliegen. Hierunter sind Suspensionen des Astaxanthin-Derivats bevorzugt, d. h. wenigstens 90 Gew.-%, insbesondere wenigstens 95 Gew.-% des Astaxanthin-Derivats liegen in suspendierter Form vor, d. h. in Form von Partikeln, die in dem Verdünnungsmittel suspendiert sind. Hierbei hat es sich als vorteilhaft erwiesen, wenn die Partikel des Astaxanthin-Derivats in der Suspension einen volumenmittleren Teilchendurchmesser (D_{4,3}-Wert) im Bereich von 0,1 bis 50 µm, insbesondere im Bereich von 0,2 bis 30 µm und speziell im Bereich von 0,5 bis 20 µm aufweisen. Unter dem volumenmittleren Teilchendurchmesser versteht man den mittels Fraunhofer-Beugung an einer verdünnten 0,01 bis 0,1 gew.-%igen, speziell 0,05 gew.-%igen Schmelze der Formulierung in flüssigen Ölen bestimmten volumenmittleren Teilchendurchmesser. Vorzugsweise weisen mindestes 90 Vol.-% der Partikel des Astaxanthin-Derivats einen Teilchendurchmesser unterhalb 100 µm, insbesondere von maximal 60 µm, besonders bevorzugt von maximal 40 µm und speziell von maximal 20 µm auf (D₉₀-Wert).

Die feste Formulierung kann grundsätzlich beliebige Formen aufweisen. Für die erfindungsgemäße Anwendung hat es sich als vorteilhaft erwiesen, wenn die die feste Formulierung eine schüttfähige, partikelförmige Formulierung ist. Hierunter versteht der Fachmann Formulierungen, die aus einer Vielzahl diskreter Partikel bestehen. In derartigen Formulierungen weisen die Partikel typischerweise Maximalabmessungen von in der Regel nicht mehr als 1 cm auf. Vorzugsweise liegen der Mittelwert (Gewichtsmittel) der Maximalabmessungen der Partikel im Bereich von 100 bis 10000 µm, insbesondere im Bereich von 200 bis 5000 µm. Vorzugsweise weisen wenigstens 90 Gew.-% der Partikel maximale Abmessungen im Bereich von 200 µm bis 10000 µm auf. Unter einer Maximalabmessung versteht man die jeweils größte Abmessung, d. h. den Abstand der jeweils am weitest voneinander entfernten Punkte auf der Oberfläche eines Partikels. Die Form der Partikel ist dabei von untergeordneter Bedeutung. Es kann sich dabei um Schuppen, kugelförmige Partikel, zylindrische Partikel, Pastillen oder unregelmäßig geformte Partikel handeln.

Gemäß einer bevorzugten Ausführungsform der Erfindung handelt es sich bei den erfindungsgemäßen partikelförmigen Formulierungen um Granulate. Vorzugsweise sind die Partikel des Granulats regelmäßig geformt. Häufig weisen sie eine kugelförmige, pastillenförmige oder zylindrische Form auf. Vorzugsweise liegt der Gewichtsmittlere Partikeldurchmesser der Partikel des Granulats im Bereich von 100 bis 5000 µm, insbesondere im Bereich von 200 bis 2000 µm. Vorzugsweise weisen wenigstens 90 Gew.-% der Partikel Partikeldurchmesser im Bereich von 200 bis 10000 µm auf.

Die partikelförmigen Formulierungen können ein Trenn- oder Rieselhilfsmittel enthalten, um ein Verkleben der Partikel zu vermeiden oder zu verringern. Bei den Rieselhilfsmitteln handelt es sich typischerweise um inerte, für Nahrungsmittel geeignete feste anorganische Materialien in Pulverform, z. B. um Oxide wie Aluminiumoxid, Siliciumdioxid, insbesondere in Form von feinteiliger Kieselsäure wie pyrogene Kieselsäure oder Kieselgel (E 551), oder Titandioxid (E 171), Hydroxide wie Aluminiumhydroxid, Carbonate und Hydrogencarbonate wie Natriumcarbonat (E 500), Natriumhydrogencarbonat, Kaliumcarbonat (E 501), Magnesiumcarbonat (E 504) und Calciumcarbonat (E 170), oder Silikate wie Natriumsilikat (E 550), Magnesiumsilikat (E 553a), Talk (E 553b), Calciumsilikat (E 552), Zinksilikat (E 557), Aluminiumsilikate wie Natriumaluminiumsilikat (554), Kaliumaluminiumsilikat (E 555), Calciumaluminiumsilikat (E 556), Bentonit (E 558), Kaolin (E 559), Tricalciumphosphat, Natriumchlorid, und/oder um inerte, für Nahrungsmittel geeignete feste organische Materialien in Pulverform, wie beispielsweise Mono-, Di- und höhere Polysacharide wie Lactose, modifizierte Lactose, Zucker (Saccharose), Cellulose, Methylcellulose, Stearinsäure oder Stearate wie Magnesiumstearat (E 572), Ammoniumstearat (E 571) und Aluminiumstearat (E 573). Vorzugsweise weisen die Mahl oder Rieselhilfsmittel volumenmittlere Teilchengrößen im Bereich von 0,5 bis 200 µm auf. Vorzugsweise ist der mittlere Partikeldurchmesser des Rieselhilfsmittels kleiner als der mittlere Durchmesser der Partikel in der partikelförmigen Formulierung, z. B. um den Faktur 2 bis 50, insbesondere um den Faktor 4 bis 20. Der Anteil der Rieselhilfsmittel wird 10 Gew.-%, bezogen auf das Gesamtgewicht der Formulierung, nicht überschreiten und beträgt, sofern vorhanden, typischerweise 0,1 bis 10 Gew.-%, vorzugsweise 0,2 bis 50 Gew.-%, insbesondere 0,3 bis 3 Gew.-%, bezogen auf das Gesamtgewicht der Formulierung.

Die erfindungsgemäßen Zusammensetzungen können auch Fließhilfsmittel enthalten. Hierbei handelt es sich um Materialien, die ein Verkleben/Verbacken der Partikel der festen Formulierung untereinander oder mit den Behälterwänden aufgrund von Fließ- oder Schmelzprozessen an den Oberflächen der Partikel der festen Formulierung verringern. Im Unterschied zu den Trenn- oder Rieselhilfsmitteln befinden sich die Fließhilfsmittel in der festen Formulierung in suspendierter Form. Bevorzugte Fließhilfsmittel sind feinteilige Kieselsäuren, insbesondere solche mit Primärpartikelgrößen im unterhalb 500 nm, wie beispielsweise sprühgetrocknete Fällungskieselsäure, z. B. die unter der Handelsmarke Sipernat® (Evonik) vertriebenen Produkte, z. B. Sipernat® 22 S, Sipernat® 50 S, Sipernat® 500 LS, pyrogene Kieselsäuren wie die unter der Handelsmarke Aerosil® (Evonik) vertriebenen Produkte, z. B. Aerosil® 200 und Aerosil® R972 sowie amorphe Kieselgele, z. B. die unter der Handelsmarke Sylox® (Grace Davison) vertriebenen Produkte wie Sylox® 2, Sylox® 15 und Sylox® T450. Die Konzentration derartiger Fließhilfsmittel liegt typischerweise im Bereich von 0,1 und 25 Gew.-%, insbesondere im Bereich von 1,0 und 10,0 Gew.-% und besonders bevorzugt im Bereich von 1,5 und 5,0 Gew.-%, bezogen auf das Gesamtgewicht der erfindungsgemäßen Formulierung.

Alternativ kann die Formulierung auch grobteilig sein, beispielsweise in Form von Platten oder Blöcken. Gegebenenfalls kann man derartige Formulierungen vor der erfindungsgemäßen Anwendung zerkleinern, um ein Lösen der Formulierungen in dem flüssigen Öl zu beschleunigen.

Zur Herstellung erfindungsgemäßer Formulierungen, in denen das Astaxanthin-Derivat in Form einer Suspension vorliegt, wird man in der Regel so vorgehen, dass man das Astaxanthin-Derivat, das zu wenigstens 70 % aus wenigstens einer Verbindung der Formel I besteht und das vorzugsweise die oben genannten Anforderungen an Reinheit und Gehalt an all-trans-Isomeren erfüllt, in einer Schmelze des für Nahrungsmittel geeigneten Verdünnungsmittels suspendiert und anschließend die dabei erhaltene flüssigen Suspension des Astaxanthin-Derivats in der Schmelze des für Nahrungsmittel geeigneten Verdünnungsmittels abkühlt.

Hierzu kommen grundsätzlich Verfahren in Betracht, bei denen ein durch Fällung oder in sonstiger Weise hergestelltes Pulver des Astaxanthin-Derivats in der Schmelze des Verdünnungsmittels suspendiert wird, sowie Verfahren, bei denen ein festes Astaxanthin-Derivat in einer Schmelze des Verdünnungsmittels durch Eintrag mechanischer Energie, vorzugsweise durch Vermahlen, auf die gewünschte Teilchengröße gebracht wird. Zur Herstellung der Suspension kann man auch eine Suspension des Astaxanthin-Derivats in einem für Nahrungsmittel geeigneten flüssigen Verdünnungsmittel, d. h. ein Verdünnungsmittel mit einem Schmelzpunkt unterhalb 40 °C, insbesondere maximal 30 °C, in eine Schmelze des festen Verdünnungsmittels einarbeiten.

Das zur Herstellung der Suspension eingesetzte Astaxanthin-Derivat weist üblicherweise eine Reinheit, bezogen auf das in Formel I gezeigten all-trans-Isomer, von wenigstens 70 Gew.-%, häufig wenigstens 80 Gew.-%, vorzugsweise wenigstens 90 Gew.-% und insbesondere wenigstens 96 Gew.-% auf. Neben dem all-trans-Isomeren kann das Astaxanthin-Derivat auch Anteile an Verbindungen der Formel I enthalten, in denen eine oder mehrere Doppelbindungen der in Formel I dargestellten all-trans-Isomere cis-Konfiguration aufweisen. Die Gesamtmenge an all-trans-Isomer der Formel I und cis-Isomeren der Formel I beträgt in der Regel wenigstens 80 Gew.-%, häufig wenigstens 90 Gew.-%, insbesondere wenigstens 96 Gew.-%. Neben dem all-trans-Isomeren der Formel I und etwaigem cis-Isomeren kann das zur Herstellung eingesetzte Astaxanthin-Derivat auch weitere Carotinoide enthalten, wobei der Anteil dieser Verunreinigungen in der Regel 20 Gew.-%, insbesondere 10 Gew.-%, besonders bevorzugt 4 Gew.-%, bezogen auf die Gesamtmenge des eingesetzten Astaxanthin-Derivats nicht überschreitet. Bei diesen Verunreinigungen handelt es sich in erster Linie um Halbester des Astaxanthins, d. h. um Verbindungen der zuvor gezeigten Formel II bzw. um cis-Isomere davon, sowie um Astaxanthin selber. Insbesondere verwendet man ein Astaxanthin-Derivat, das die an ein für Nahrungsmittel (d. h. für Lebens- und/oder Futtermittel) zugelassenes Astaxanthin gestellten Anforderungen erfüllt.

Naturgemäß erfolgt das Suspendieren des Astaxanthin-Derivats in die Schmelze, d. h. bei einer Temperatur oberhalb des Schmelzpunkts des Verdünnungsmittels. In der Regel wird die Temperatur etwa 1 bis 50 K, insbesondere 5 bis 40 K oberhalb des Schmelzpunktes des festen Verdünnungsmittels liegen und vorzugsweise eine Temperatur von 110 °C, insbesondere 100 °C nicht oder nur kurzfristige während des Suspendierens überschreiten.

Das zur Herstellung der Suspension eingesetzte Verdünnungsmittel kann Antioxidantien, z. B. Tocopherole wie α-Tocopherol oder Ascorbylpalmitat und/oder lipophile Dispergiermittel, vorzugsweise in den oben angegebenen Mengen, enthalten. Daneben kann das Verdünnungsmittel auch geringe Mengen an emulgiertem oder gelöstem Wasser, vorzugsweise jedoch nicht mehr als 10 Gew.-%, insbesondere nicht mehr als 1 Gew.-%, bezogen auf die Gesamtmenge des eingesetzten Verdünnungsmittel enthalten.

Gemäß einer bevorzugten Ausführungsform erfolgt die Bereitstellung der Suspension durch Vermahlen des Astaxanthin-Derivats, welches die oben genannte Reinheit aufweist, in einer Schmelze des Verdünnungsmittels. Hierzu wird man in der Regel das Astaxanthin-Derivat zunächst in der Schmelze des Verdünnungsmittels suspendieren, wobei man eine grobteilige Suspension des Astaxanthin-Derivats erhält, und anschließend die Partikel in einer geeigneten Vorrichtung zum Vermahlen auf die gewünschte Teilchengröße zerkleinern. Als Vorrichtungen zum Vermahlen können übliche, dem Fachmann bekannte Vorrichtungen, beispielsweise Kugelmühlen, Perlmühlen oder Kolloid-Mühlen eingesetzt werden, die gegebenenfalls beheizbar sind, um die für das Schmelzen des Verdünnungsmittels notwendigen Temperaturen zu gewährleisten.

Das Vermahlen erfolgt typischerweise bei Temperaturen unterhalb 110 °C, häufig im Bereich von 50 bis 100 °C und insbesondere im Bereich von 60 bis 90 °C.

Das Vermahlen wird solange durchgeführt, bis die gewünschte Teilchengröße erreicht ist. Vorzugsweise wird die Vermahlung solange fortgesetzt, bis die Partikel des Astaxanthin-Derivats in der Suspension einen volumenmittleren Teilchendurchmesser im Bereich von 0,1 bis 50 µm, insbesondere im Bereich von 0,2 bis 30 µm und speziell im Bereich von 0,5 bis 20 µm aufweisen.

Anschließend wird man die noch flüssige, d. h. geschmolzene, Suspension abkühlen, wobei man die erfindungsgemäße feste Formulierung des Astaxanthin-Derivats erhält, in der das Astaxanthin-Derivat im Wesentlichen suspendiert in dem festen Verdünnungsmittel vorliegt. Die so erhaltene feste Formulierung des suspendierten Astaxanthin-Derivats kann auch gelöste Anteile des Astaxanthin-Derivats enthalten, wobei der gelöste Anteil in der Regel weniger als 50 Gew.-%, vorzugsweise weniger als 25 Gew.-%, insbesondere weniger als 10 Gew.-% oder weniger als 5 Gew.-% des in der Formulierung enthaltenen Astaxanthin-Derivats ausmacht.

Das Abkühlen der noch flüssigen Suspension des Astaxanthin-Derivats kann grundsätzlich in beliebiger Weise erfolgen, wobei ein rasches Abkühlen bevorzugt ist, um einen prinzipiell möglichen Abbau des Astaxanthin-Derivats weitestgehend zu verhindern. Beispielsweise kann man die noch flüssige Suspension durch Ausgießen auf gekühlte Walzen oder Platten abschrecken, wobei sich eine feste Schicht bildet, die anschließend zerkleinert werden kann. Alternativ kann man auch ein so genanntes Prilling, Jet-Prilling oder Kryoprilling der flüssigen Suspension durchführen. Hierzu wird man die flüssige Suspension in Tröpfchen überführen, die man mittels eines kühlen Gasstroms, beispielsweise mittels eines kühlen Stickstoffstroms, oder einer kühlen Flüssigkeit, in der das Verdünnungsmittel unlöslich ist, beispielsweise Wasser, abschreckt. Ebenfalls ist es möglich, so genannte Pastillierverfahren anzuwenden, bei denen man typischerweise die flüssige Suspension auf gekühlte Flächen, z. B. gekühlte rotierende Teller oder Platten tropft und anschließend von der gekühlten Fläche mittels geeigneter Vorrichtungen entfernt.

Alternativ kann man zunächst eine Suspension des Astaxanthin-Derivats in einem flüssigen Verdünnungsmittel herstellen und diese Suspension in eine Schmelze des festen Verdünnungsmittels einarbeiten. Hierbei ist es ausreichend, wenn in der daraus resultierenden Suspension das Verdünnungsmittel, also die Mischung des flüssigen Verdünnungsmittels mit dem festen Verdünnungsmittel, die erfindungsgemäßen Eigenschaften aufweist. Insbesondere hat es sich bewährt, die Suspension in einem niedriger schmelzenden, für Nahrungsmittel geeigneten Lösungsmittel, insbesondere einem für Nahrungsmittel geeigneten Öl herzustellen, das einen Schmelzpunkt bei Normaldruck unterhalb 30 °C aufweist. Beispiele hierfür sind Pflanzenöle wie Sojabohnenöl, Palmöl, Palmkernöl, Sonnenblumenöl, Distelöl, Maiskeimöl, Olivenöl, Leinsamenöl, Rapsöl, Reisöl, Kokosöl, Erdnussöl, PUFA-Öle, Triglyceride mittelkettiger (= C₈-C₁₀) pflanzlicher Fettsäuren (sog. MCT-Öle), semisynthetische Triglyceride, z. B. Caprylsäure/Caprinsäure-Tricyceride wie die Miglyol-Typen, weiterhin Fischöle, sowie Mischungen dieser Öle. In der Suspension werden die Partikel des Astaxanthin-Derivats vorzugsweise einen volumenmittleren Teilchendurchmesser im Bereich von 0,1 bis 50 µm, insbesondere im Bereich von 0,2 bis 30 µm und speziell im Bereich von 0,5 bis 20 µm aufweisen. Das Einarbeiten erfolgt durch Vermischen der Suspension mit der Schmelze des festen Verdünnungsmittels bei den zuvor genannten Temperaturen.

Zur Herstellung von Lösungen des Astaxanthin-Derivats wird man eine Suspension des Astaxanthin-Derivats in der Schmelze des Verdünnungsmittels so lange erhitzen, bis das Astaxanthin-Derivat weitgehend oder vollständig gelöst vorliegt. Ein vollständiges Lösen ist in der Regel nicht erforderlich, da die Lösungen im kalten Zustand stabil sind und das Astaxanthin-Derivat in dem festen Verdünnungsmittel nicht rekristallisiert. In der Regel liegen in den Lösungen wenigstens 50 Gew.-%, insbesondere wenigstens 80 Gew.-% oder wenigstens 90 Gew.-%, speziell wenigstens 98 Gew.-% des in der Formulierung enthaltenen Astaxanthin-Derivats in gelöster Form vor.

Hierbei wird man in der Regel so vorgehen, dass man zunächst eine Suspension des Astaxanthin-Derivats in einem ersten, für Nahrungsmittel geeigneten Verdünnungsmittel herstellt und diese Suspension in der Schmelze des festen Verdünnungsmittel erhitzt, bis eine Lösung des Astaxanthins in der Schmelze vorliegt. Es versteht sich von selber, dass das erste Verdünnungsmittel nicht notwendigerweise ein festes Verdünnungsmittel im Sinne dieser Erfindung sein muss. Vielmehr ist es ausreichend, wenn in der daraus resultierenden Lösung das Verdünnungsmittel, also die Mischung des ersten Verdünnungsmittels mit dem festen Verdünnungsmittel, die erfindungsgemäßen Eigenschaften aufweist. Dementsprechend können das in der Suspension enthaltene Verdünnungsmittel und das geschmolzene feste Verdünnungsmittel in ihrer Zusammensetzung identisch sein, sind es aber nicht notwendigerweise. Beispielsweise kann das zur Herstellung der Suspension einen niedrigeren Schmelzpunkt oder höhere Polarität aufweise als geschmolzene feste Verdünnungsmittel.

Insbesondere hat es sich bewährt, die Suspension in einem niedriger schmelzenden, für Nahrungsmittel geeigneten Verdünnungsmittel, insbesondere einem für Nahrungsmittel geeigneten Öl herzustellen, dass einen Schmelzpunkt bei Normaldruck unterhalb von 40 °C, insbesondere von maximal 30 °C aufweist. Beispiele hierfür sind Pflanzenöle wie Sojabohnenöl, Palmöl, Palmkernöl, Sonnenblumenöl, Distelöl, Maiskeimöl, Olivenöl, Leinsamenöl, Rapsöl, Reisöl, Kokosöl, Erdnussöl, PUFA-Öle, Triglyceride mittelkettiger (= C₈-C₁₀) pflanzlicher Fettsäuren (sog. MCT-Öle), semisynthetische Triglyceride, z. B. Caprylsäure/Caprinsäure-Tricyceride wie die Miglyol-Typen, weiterhin Fischöle, sowie Mischungen dieser Öle. In der Suspension werden die Partikel des Astaxanthin-Derivats vorzugsweise einen volumenmittleren Teilchendurchmesser im Bereich von 0,1 bis 50 µm, insbesondere im Bereich von 0,2 bis 30 µm und speziell im Bereich von 1 bis 20 µm aufweisen.

Vorzugsweise erfolgt das Lösen des Astaxanthins in der Schmelze des Verdünnungsmittels durch rasches Erhitzen in an sich bekannter Weise, z. B. durch Wärmetauscher, oder in sonstiger Weise. In einer bevorzugten Ausgestaltung erfolgt das Erhitzen durch Vermischen der Suspension, die das Astaxanthin-Derivat in einer Teilmenge des benötigten, geschmolzenen Verdünnungsmittels oder einem anderen für Nahrungsmittel geeigneten Verdünnungsmittel enthält, mit weiterem geschmolzenen Verdünnungsmittel, das eine höhere Temperatur aufweist als die Suspension, wobei der Temperaturunterschied so gewählt ist, dass in der Mischung die zum Lösen erforderliche Temperatur resultiert. In der Regel liegt die Temperatur in der so erhaltenen Mischung im Bereich von 50 bis 220 °C, vorzugsweise im Bereich von 70 bis 200 °C und insbesondere im Bereich von 90 bis 180 °C. Die Temperaturdifferenz zwischen der Suspension und dem geschmolzenen Verdünnungsmittel beträgt in der Regel wenigstens 20 K und insbesondere wenigstens 40 K, z. B. 20 bis 220 K und insbesondere 40 bis 200 K oberhalb derjenigen Temperatur, welche die Carotinoid-Suspension vor dem Vermischen mit der Schmelze des Verdünnungsmittels aufweist.

Das Vermischen der geschmolzenen Suspension mit der Schmelze des weiteren Verdünnungsmittels kann in üblicher Weise erfolgen. Vorzugsweise wird man einen Strom der geschmolzenen Suspension mit einem Strom des geschmolzenen weiteren Verdünnungsmittels in einer kontinuierlich arbeitenden Mischvorrichtung, die beispielsweise ein statischer Mischer oder eine Pumpe sein kann, vereinigen und den so erhaltenen heißen Strom, gegebenenfalls nach Durchlaufen einer vorzugsweise beheizten oder wärmeisolierten Haltestrecke zum Lösen des Astaxanthin-Derivats, abkühlen.

Die so erhaltene heiße Lösung wird dann abgekühlt, wobei man eine feste Formulierung enthält, in der in der Regel wenigstens 50 Gew.-%, insbesondere wenigstens 80 Gew.-% oder wenigstens 90 Gew.-%, speziell wenigstens 98 Gew.-% des in der Formulierung enthaltenen Astaxanthin-Derivats in gelöster Form vorliegt.

Das Abkühlen der noch flüssigen Lösung des Astaxanthin-Derivats kann grundsätzlich in der für die geschmolzene Suspension beschriebenen Weise erfolgen, wobei ein rasches Abkühlen bevorzugt ist, um einen prinzipiell möglichen Abbau des Astaxanthin-Derivats weitestgehend zu verhindern. Insbesondere bieten sich hierfür die vorgenannten Verfahren, wie das Abschrecken durch Aufgießen auf gekühlte Walzen, das Prilling, einschließlich Jetprilling oder Kryoprilling, sowie Pastillierverfahren an.

Vorzugsweise erfolgt das Lösen der Suspension in der Schmelze des Verdünnungsmittels möglichst rasch, so dass die mittlere Verweilzeit der dabei erhaltenen heißen Lösung in der zum Erhitzen verwendeten Vorrichtung bis zum Abkühlen/Abschrecken möglichst kurz ist und eine Dauer von 30 Sekunden, insbesondere 20 Sekunden, bevorzugt 10 Sekunden und speziell 5 Sekunden nicht überschreitet. Typischerweise wird die mittlere Verweilzeit der heißen Lösung bis zum Abkühlen/Abschrecken 0,1 bis 30 Sekunden, insbesondere 0,1 bis 20 Sekunden, bevorzugt 0,1 bis 10 Sekunden und speziell 0,2 bis 5 Sekunden oder 0,5 bis 5 Sekunden betragen.

Die erfindungsgemäßen festen Formulierungen lassen sich einfach in flüssige, für Nahrungsmittel geeignete Öle einarbeiten und eigenen sich daher in besondere Weise zur Herstellung von Futtermittelzubereitungen, insbesondere festen Futtermittelzubereitungen, speziell Futtermittelzubereitungen in Form von Pellets.

Dementsprechend betrifft die vorliegende Erfindung auch die Verwendung der erfindungsgemäßen Formulierungen zur Herstellung von Lösungen des Astaxanthin-Derivats in für Nahrungsmittel geeigneten Ölen.

Dementsprechend betrifft die vorliegende Erfindung auch ein Verfahren zur Herstellung von Futtermittelzubereitungen, insbesondere von festen Futtermittelzubereitungen, speziell von Pellets, umfassend die folgenden Schritte:
a) Bereitstellung einer erfindungsgemäßen Formulierung wie hier beschrieben;
b) Lösen der Formulierung mit einem für Nahrungsmittel geeigneten, unter Prozessbedingungen flüssigen Öl unter Erhalt einer verdünnten flüssigen Lösung des Astaxanthin-Derivats in dem flüssigen Öl; und
c) Vermischen der in Schritt b) erhaltenen flüssigen Lösung mit den Bestandteilen der Futtermittelzubereitung oder Imprägnieren einer festen Futtermittelzubereitung mit der in Schritt b) erhaltenen flüssigen Lösung.

Unter "Prozessbedingungen flüssig" bedeutet, dass das für Nahrungsmittel geeignete Öl unter den Bedingungen des Einarbeitens der in Schritt b) erhaltenen Lösung in die Bestandteile der Futtermittelzusammensetzung in Schritt c) flüssig ist.

Das in Schritt b) eingesetzte Öl kann synthetischer, mineralischer, pflanzlicher oder tierischer Herkunft sein. Beispiel sind Pflanzenöle wie Sojabohnenöl, Sonnenblumenöl, Maiskeimöl, Leinsamenöl, Rapsöl, Distelöl, Weizenkeimöl, Reisöl, Kokosöl, Mandelöl, Aprikosenkernöl, Palmöl, Palmkernöl, Avocadoöl, Jojobaöl, Haselnussöl, Walnussöl, Erdnussöl, Pistazienöl, Triglyceride mittelkettiger (= C₈-C₁₀) pflanzlicher Fettsäuren (sog. MCT-Öle) und PUFA-Öle (PUFA = mehrfach ungesättigte Fettsäuren (polyunsaturated fatty acids) wie Eikosapentaensäure (EPA), Docosahexaensäure (DHA) und α-Linolensäure), weiterhin semisynthetische Triglyceride, z. B. Caprylsäure/Caprinsäure-Tricyceride wie die Miglyol-Typen, weiterhin Paraffinöl, flüssige hydrierte Polyisobutene, Squalan, Squalen, weiterhin tierische Öle und Fette wie Fischöle einschließlich Makrelen-, Sprotten-, Thunfisch-, Heilbutt-, Kabeljau- und Lachsöl.

Bevorzugt sind pflanzliche Öle und Öle tierischen Ursprungs, die bei 40 °C flüssig sind, insbesondere Pflanzenöle wie Sojabohnen-Öl, Sonnenblumenöl, Distelöl, Maiskeimöl, Olivenöl, Leinsamenöl, Rapsöl, Reisöl, Kokosöl, Erdnussöl, Palmöl, Palmkernöl, PUFA-Öle, MCT-Öle, weiterhin Fischöle, sowie Mischungen dieser Öle.

In einer bevorzugten Ausführungsform der Erfindung umfasst das in Schritt b) eingesetzte Öl zu wenigstens 50 Gew.-% und insbesondere zu wenigstens 80 Gew.-%, bezogen auf die Gesamtmenge des in Schritt b) eingesetzten essbaren Öls, wenigstens ein unter Maisöl, Sonnenblumenöl, Sojabohnenöl und Fischöl ausgewähltes essbares Öl.

Bei den in Schritt b) eingesetzten Ölen kann es sich um Raffinatöle oder Rohöle handeln, die noch ursprungsspezifische Verunreinigungen wie Proteine, Phosphat, (Erd-)Alkalimetallsalze und dergleichen in üblichen Mengen enthalten. Die in Schritt b) eingesetzten Öle können auch geringe Mengen an emulgiertem oder gelöstem Wasser, vorzugsweise jedoch nicht mehr als 10 Gew.-%, insbesondere nicht mehr als 1 Gew.-%, bezogen auf die Gesamtmenge des in Schritt b) eingesetzten Öls, enthalten.

Das Vermischen des flüssigen Öls mit der festen Formulierung erfolgt in der Regel bei Temperaturen oberhalb des Schmelzpunktes des in der festen Formulierung enthaltenen flüssigen Verdünnungsmittels, wobei in der Regel eine Temperatur ausreicht die wenigstens 1 K, insbesondere wenigstens 5 K oberhalb des Schmelzpunktes des in der festen Formulierung enthaltenen flüssigen Verdünnungsmittels liegt. In der Regel wird man beim Einarbeiten der festen Formulierung in das flüssige Öl eine Temperatur unterhalb 100 °C einhalten. Das Einarbeiten der erfindungsgemäßen Formulierung in das flüssige Öl erfolgt typischerweise bei Temperaturen von maximal 100 °C, vorzugsweise bei Temperaturen von nicht mehr als 95 °C und speziell nicht mehr als 90 °C und vorzugsweise bei Temperaturen im Bereich von 45 bis 100 °C, insbesondere im Bereich von 55 bis 95 °C und speziell im Bereich von 60 bis 90 °C.

Vorzugsweise wird man das Öl, in welches man die feste Formulierung des Astaxanthin-Derivats einarbeitet, erwärmen. In der Regel liegt die Temperatur des erwärmten Öls vorzugsweise maximal 100 °C und beträgt insbesondere nicht mehr als 95 °C und speziell nicht mehr als 90 °C, und liegt vorzugsweise im Bereich von 45 bis 100 °C, insbesondere im Bereich von 50 bis 95 °C und speziell im Bereich von 55 bis 90 °C.

Typischerweise wird man die relativen Mengen an Öl und fester Formulierung so wählen, dass der Gehalt an Astaxanthin-Derivat in der resultierenden Lösung im Bereich von 10 ppm (= 0,001 Gew.-%) bis etwa 1 Gew.-%, häufig im Bereich von 20 ppm bis 0,5 Gew.-%, insbesondere im Bereich von 50 ppm bis 0,2 Gew.-%, bezogen auf die Gesamtmenge an Öl plus Astaxanthin-Derivat liegt.

Beispielsweise kann man so vorgehen, dass man ein für Nahrungsmittel geeignetes Öl, das vorzugsweise einen Schmelzpunkt von nicht mehr als 40 °C aufweist, in einem geeigneten Gefäß vorlegt, gegebenenfalls auf die gewünschte Temperatur vorwärmt und hierzu die erfindungsgemäße Formulierung unter Durchmischen zugibt, wobei die Zugabe in einer Portion, in mehreren Portionen oder kontinuierlich erfolgen kann. Die zugegebene feste Formulierung des Astaxanthin-Derivats kann auf die gewünschte Temperatur vorgewärmt werden, was jedoch grundsätzlich nicht erforderlich ist.

Das Einarbeiten der erfindungsgemäßen Formulierung in das für Nahrungsmittel geeignete Öl kann auch kontinuierlich durch so genannte Inline-Mischer, d. h. kontinuierlich arbeitende Mischvorrichtungen, z. B. statische Mischer oder dynamische Mischer erfolgen. Beispiele für geeignete statische Mischer sind Mischkammem mit oder ohne Einbauten, Mischrohre mit oder ohne Mischdüsen, Jet-Mischer und der gleichen. Beispiele für dynamische Mischer sind Rotor-Stator-Mischer, Mischkammem mit Rührwerken, Mischpumpen und dergleichen. In diesem Fall wird man die feste Formulierung aufschmelzen. Gegebenenfalls schließt sich der Mischvorrichtung noch eine Verweilzone an, die gegebenenfalls temperiert werden kann, um das Lösen des Astaxanthin-Derivats zu vervollständigen.

Die zum Erreichen des Lösens erforderliche Mischdauer hängt naturgemäß von der Temperatur, dem gewählten Öl und den apparativen Gegebenheiten ab und liegt typischerweise im Bereich von 10 sec. bis 120 min. Der Fachmann kann die erforderliche Mischzeit durch Routineexperimente ermitteln.

Im Anschluss an das Mischen kann man die erhaltene ölige Lösung, sofern erforderlich abkühlen, z. B. mittels geeigneter Wärmetauscher oder durch Verdünnen mit einem kalten essbaren Öl, das zu dem zum Lösen in Schritt b) eingesetzten Öl gleich oder verschieden sein kann.

Auf diese Weise erhält man stabile Lösungen von Astaxanthin-Derivaten in für Nahrungsmittel geeigneten Ölen. Diese Lösungen zeichnen sich dadurch aus, dass das in ihnen enthaltene Astaxanthin-Derivat einen hohen Anteil an all-trans-Isomeren aufweist, der in der Regel oberhalb 80 %, insbesondere oberhalb 90 % liegt.

Die so erhaltenen Lösungen enthalten neben dem Astaxanthin-Derivat die in der Formulierung enthaltenen Hilfsstoffe, wie ggf. Oxidationsstabilisatoren, lipophile Dispergiermittel, Fließhilfsmittel und/oder Verdicker.

Es versteht sich von selber, dass die in Schritt b) erhaltenen Lösungen geringe Mengen an Wasser aus dem zu ihrer Herstellung eingesetzten, für Nahrungsmittel Öl enthalten können. Häufig jedoch beträgt der Wasseranteil nicht mehr als 10 Gew.-%, z. B. 0,1 bis 10 Gew.-%, häufig 0,5 bis 8 Gew.-%, und in einer speziellen Ausführungsform der Erfindung nicht mehr als 0,5 Gew.-%, bezogen auf das in der Lösung enthaltene Öl.

Die nach dem erfindungsgemäßen Verfahren erhältlichen Lösungen des Astaxanthin-Derivats sind lagerstabil und können vor ihrer weiteren Verwendung über längere Zeiträume aufbewahrt werden, ohne dass es in nennenswertem Umfang zu einem Aktivitätsverlust, z. B. durch Isomerisierung und/oder oxidativen Abbau kommt. Insbesondere zeichnen sie sich durch einen hohen Anteil an all-trans-Isomeren des eingesetzten Astaxanthin-Derivats aus und durch einen vergleichsweise geringen Anteil an Abbauprodukten des Astaxanthin-Derivats.

Das erfindungsgemäße Verfahren wird häufig direkt in die Prozesse zur Weiterverarbeitung der Lösungen des Astaxanthin-Derivats integriert werden.

Die nach dem erfindungsgemäßen Verfahren erhältlichen Lösungen des Astaxanthin-Derivats eignen sich in vorteilhafter Weise als Zusatz zu Tierfuttermitteln, Nahrungsmitteln für die Humanernährung, Nahrungsergänzungsmitteln, pharmazeutischen Mitteln oder kosmetischen Mitteln. Bevorzugt lassen sich die Lösungen als Futtermittelzusatz in der Tieremährung einsetzen, beispielsweise bei der Futtermittelherstellung durch Einmischen in einen Futtermittelteig vor oder während der Extrusion oder durch Auftragen bzw. Aufsprühen auf Futtermittelpellets. Die Anwendung als Futtermittelzusatz erfolgt insbesondere durch direktes Aufsprühen der erfindungsgemäßen Formulierungen, beispielsweise als sogenannte "post-pelleting liquid application". Vorzugsweise belädt man die Futtermittelpellets mit den Formulierungen unter vermindertem Druck.

Dementsprechend betrifft die vorliegende Erfindung auch die Herstellung von Futtermitteln, wie Tierfuttermittel, Nahrungsmittel für die Humanernährung, Nahrungsergänzungsmittel, sowie die Herstellung pharmazeutischer Mittel oder kosmetischer Mittel unter Verwendung der erfindungsgemäß hergestellten Lösungen des Astaxanthin-Derivats. Diese Mittel enthalten neben den für diese Mittel üblichen Bestandteilen das bei 30 °C flüssige Öl, das feste Verdünnungsmittel und das Astaxanthin-Derivat.

Bevorzugte Ausführungsformen betreffen Tierfuttermittel, insbesondere Fischfuttermittel, die das flüssige Öl, das feste Verdünnungsmittel und das Astaxanthin-Derivat umfassen. Derartige Mittel enthalten das in der Lösung enthaltene Astaxanthin-Derivat typischerweise in einer Menge von 10 bis 100 ppm, bezogen auf das Gesamtgewicht des Mittels, wobei das Astaxanthin-Derivat in der Regel zu mehr als 70 %, insbesondere zu wenigstens 80 % und speziell zu wenigstens 90 % eine all-trans-Konfiguration aufweist.

Typische Bestandteile in Futtermitteln sind Kohlehydrat-Quellen, insbesondere Getreidemehle wie Weizen- oder Maismehl, Sojabohnenmehl, aber auch Zucker und Zuckeralkohole, weiterhin proteinhaltige Bestandteile wie Sojakonzentrat, Fischmehl, Glutene wie Mais- oder Weizengluten, Öle und Fette, z. B. die zuvor genannten essbaren Öle, aber auch andere essbare Fette pflanzlichen oder tierischen Ursprungs, weiterhin Nutrazeutika wie freie Aminosäuren, deren Salze, Vitamine und Spurenelemente, sowie gegebenenfalls Verarbeitungshilfsmittel, z. B. Gleitmittel, Antiblockmittel, inerte Füllstoffe und dergleichen, und gegebenenfalls Konservierungsmittel. Typische Fischfutterzusammensetzungen enthalten z. B. Getreidemehl in einer Menge von beispielsweise 3 bis 20 Gew.-%, Gluten, z. B. in einer Menge von 1 bis 30 Gew.-%, ein oder mehrere Proteinquellen, z. B. Sojakonzentrat und/oder Fischmehl, z. B. in einer Gesamtmenge von 10 bis 50 Gew.-%, Fette und/oder Öle in einer Menge von beispielsweise 10 bis 50 Gew.-%, gegebenenfalls ein oder mehrere Vitamine in einer Gesamtmenge von beispielsweise 0,1 bis 2 Gew.-% und gegebenenfalls Aminosäuren in einer Menge von beispielsweise 0,1 bis 5 Gew.-%, jeweils bezogen auf die Gesamtmenge der Futtermittelbestandteile.

Eine spezielle Ausführungsform dieser Mittel betrifft Futtermittelpellets, speziell Futtermittelpellets für Fischfutter, die mit der erfindungsgemäß erhältlichen Lösung des Astaxanthin-Derivats beladen sind. Derartige Pellets enthaltend das in der Lösung enthaltene Astaxanthin-Derivat typischerweise in einer Menge von 10 bis 100 ppm, bezogen auf das Gesamtgewicht des Futtermittels. Die Herstellung solcher Pellets erfolgt in der Regel durch Besprühen konventioneller Pellets mit einer erfindungsgemäß erhältlichen Lösung des Astaxanthin-Derivats, vorzugsweise unter vermindertem Druck, wobei das Besprühen kontinuierlich oder vorzugsweise diskontinuierlich erfolgen kann. Beispielsweise kann man die konventionellen Pellets in einem geeigneten Gefäß vorlegen, das Gefäß evakuieren und dann Öl unter Durchmischen der Pellets aufsprühen und anschließend belüften. Auf diese Weise erreicht man ein gleichmäßiges Eindringen der erfindungsgemäßen öligen Zusammensetzung in die Pellets. Gegebenfalls kann man erneut Vakuum anlegen und erneut die Lösung des Astaxanthin-Derivats oder ein für Nahrungsmittel geeignetes, flüssiges Öl in der zuvor beschriebenen Weise aufsprühen. Auf diese Weise erhält man Pellets, die im Kern das Öl und das Astaxanthin-Derivat enthalten.

Die folgenden Beispiele dienen der weiteren Erläuterung der Erfindung.

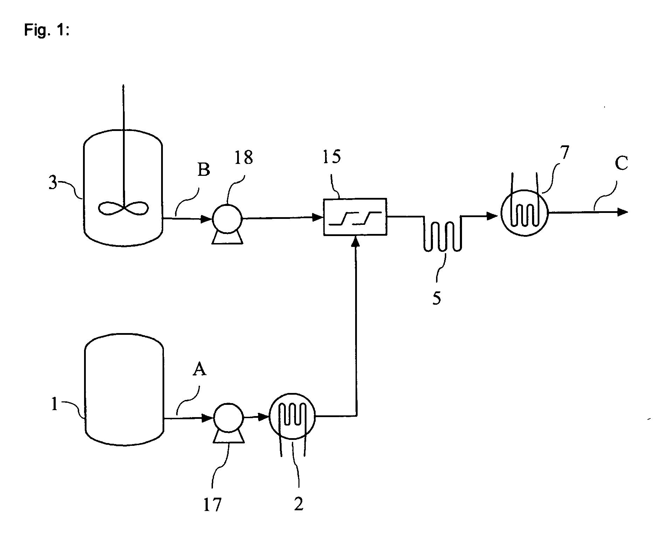

Die Bezugszeichen in den Figuren 1 und 2 haben folgende Bedeutungen:
- (1): beheizter Vorlagenbehälter für Verdünnungsmittel
- (2): Wärmetauscher
- (3): Vorlagenbehälter für Suspension des Astaxanthin-Derivats (z. B. Ax-DMDS)
- (5): Verweilzeitstrecke
- (7): Wärmetauscher
- (15): Mischvorrichtung, bzw. statischer Mischer
- (17): Förderpumpe
- (18): Förderpumpe

- A: Strom geschmolzenes Verdünnungsmittel
- B: Suspensionsstrom
- C: Strom Lösung des Astaxanthin-Derivats (Produktstrom)

Figur 1 zeigt schematisch ein semikontinuierliches Verfahren zur Herstellung einer Lösung von Ax-DMDS in einem für Nahrungsmittel geeigneten Verdünnungsmittels durch Erhitzen einer Suspension von Ax-DMDS in einer Schmelze des für Nahrungsmittel geeigneten Verdünnungsmittel, bei der das Erhitzen mittels eines heißen Stroms des geschmolzenen Verdünnungsmittels vorgenommen wird.

Gemäß dem in Figur 1 dargestellten Verfahren wird eine Schmelze eines für Nahrungsmittel geeigneten Verdünnungsmittels aus dem beheizten Vorlagenbehälter (1) mittels einer Förderpumpe (17) in den Wärmetauscher (2) geleitet und dort auf die zum Erhitzen erforderliche Temperatur T_{A} erhitzt, insbesondere auf Temperaturen im Bereich von 160 bis 230 °C. Parallel hierzu wird aus dem Vorlagenbehälter (3), welcher Mittel zum Bewegen der Suspension wie Rührer, etc. aufweist, ein Strom B einer Ax-DMDS-Suspension, die eine Temperatur T_{B} aufweist, mittels einer Förderpumpe (18) in den statischen Mischer (15) geleitet und dort mit dem auf die Temperatur T_{A} erhitzten Strom zusammengeführt. Hierbei stellt sich in der Mischung (= Strom AB), abhängig vom Verhältnis der Volumenströme A und B die Mischtemperatur T_{AB} ein, die typischerweise im Bereich von 120 bis 200 °C, insbesondere im Bereich von 150 bis 190 °C liegt. Konzentration im Strom AB liegt vorzugsweise im Bereich von 0,2 bis 10 g/kg. Das Volumenstromverhältnis von Strom A zu Strom B wird vorzugsweise im Bereich von 100 : 1 bis 1 : 1 und insbesondere im Bereich von 50 : 1 bis 2 : 1 liegen. Die auf die Temperatur T_{AB} erhitzte Mischung AB passiert die wärmeisolierter Haltestrecke (5) und wird von dort im Wärmetauscher (7) auf eine Temperatur im Bereich von 20 bis 70 °C abgekühlt und als Produktstrom C ausgeschleust. Der auf die Temperatur T_{AB} erhitzte Mischung AB passiert eine wärmeisolierter Haltestrecke (5) und wird von dort in den Wärmetauscher (16) geleitet und auf eine Temperatur 5 bis 20 K oberhalb des Schmelzpunkts des Fetts gekühlt und von dort in eine Vorrichtung zur Konfektionierung, z. B. eine gekühlte Platte, eine Prillingvorrichtung oder eine Pastilliervorrichtung geleitet (nicht gezeigt).

### Analytik:

Die Bestimmung der Partikelgrößen erfolgte mittels Fraunhoferbeugung unter Verwendung eines Malvern Mastersizer S bei 22 °C. Die durch Vermahlen erhaltenen Suspensionen des Astaxanthin-Derivats wurden vor der Messung mit Hexan auf eine Konzentration von 0,05 Gew.-% verdünnt. Angegeben ist der volumenmittlere Teilchendurchmesser D_{4,3}.

Zur Bestimmung des absoluten Gehalts an Astaxanthin-Derivat in den zur Herstellung der Lösungen verwendeten Suspensionen löste man die Suspension in Aceton und bestimmte anschließend mittels UV/VIS-Spektroskopie (200 bis 800 nm) die Extinktion der Lösung bei 478 nm.

Zur Bestimmung des Gehalts an gelöstem Astaxanthin in den erfindungsgemäß erhältlichen Formulierung, filtrierte man die geschmolzene Probe über einen Filter mit einer Porenweite von 0,2 µm und bestimmte anschließend mittels UV/VIS-Spektroskopie (200 bis 800 nm) die Extinktion der Lösung bei 478 nm.

Die Bestimmung des Anteils an all-trans-Isomer erfolgte mittels HPLC.

In den folgenden Versuchen wurde das in Beispiel 5 der WO 03/066583 beschriebene (all-E)-3,3'-rac-Astaxanthindimethyldisuccinat (Ax-DMDS) in kristalliner Form mit einer Reinheit von > 96 % eingesetzt (Verbindung der Formel I, worin R für C(=O)CH₂CH₂C(=O)OCH₃ steht).

Zur Herstellung der Suspensionen und Lösungen wurden das folgende handelsübliche Hartfett eingesetzt: Hydriertes Palmöl (Schmelzpunkt 55-57 °C, Säurezahl < 0,5, Epoxidzahl < 1: Edenor HPA der Fa. Cognis).

Zur Herstellung der Suspensionen wurde folgendes Öl eingesetzt: MCT -Öl: Delios® MCT-Öl der Fa. Cognis GmbH; Wassergehalt < 0,1 %, Gehalt an C8/C10-Fettsäuretriglyceriden > 95 %.

Allgemeine Vorschrift für das Vermahlen von Ax-DMDS

300 g Ax-DMDS werden bei 65 °C mit 2700 g einer Schmelze des Hartfetts vermischt so dass man eine 10 gew.-%ige Primärsuspension erhält. Diese Primärsuspension wird anschließend mit 4 Passagen bei Temperaturen im Bereich von 65 bis 70 °C, mit einem Druck von 0,5 bar, einer Durchflussrate von 60 bis 70 g/min in einer Kugelmühle (Drais PML1 AV: Sieb 0,4 mm, 3270 U/min, Mahlraumvolumen 1000 ml, gefüllt mit ZrO₂-stablisierten Kugeln mit einem Durchmesser von 0,7 bis 1,2 mm) vermahlen. Der Teilchendurchmesser wird nach 0,5 h und 4 h bestimmt. Nach 4 h wird die geschmolzene Suspension über die Mühle ausgefahren. Anschließend wird die Schmelze auf eine gekühlte Platte gegeben, wo sie erstarrt. Nach einer Mahldauer von 4 h liegt der volumenmittlere Partikeldurchmesser in der Regel im Bereich von 0,9 bis 1,5 µm.

Allgemeine Vorschrift für das Herstellen von Lösungen von Ax-DMDS in Fett
1. 32 g Ax-DMDS werden mit 368 g MCT-Öl (Delios der Fa. Cognis) vermischt so dass man eine 8 gew.-%ige Primärsuspension erhält. Diese Primärsuspension wird 6 h in einer konventionellen Schüttelmühle (RedDevil®) vermahlen. Die erhaltene Suspension weist einen mittleren Partikeldurchmesser d_{4,3} im Bereich von 1 bis 10 µm auf.
2. Die in Schritt 1 erhaltene Suspension von Ax-DMDS in MCT-Öl wird in der in Figur 1 gezeigten Anordnung in dem Hartfett gelöst. Hiezu wird geschmolzenes Hartfett aus dem beheizten Vorlagenbehälter (1) mittels einer Zahnradpumpe (17) mit einer Geschwindigkeit von 3,57 kg/h gefördert und mittels eines Ölbades (2) auf 220 °C erhitzt. Parallel hierzu wird aus dem Vorlagenbehälter (3) ein Strom B der in Schritt 1 hergestellten Suspension, die eine Temperatur von 22 °C aufweist, mittels einer Zahnradpumpe (18) mit einer Geschwindigkeit von 0,94 kg/h gefördert. Die Ströme A und B werden in einem ersten T-Stück als statischer Mischer (15) zusammengeführt und dabei intensiv durchmischt. Das T-Stück weist in allen Rohren einen Innendurchmesser von 0,75 mm auf. Hierbei stellte sich in der Mischung (= Strom AB) die Mischtemperatur von etwa 173 bis 175 °C ein. Die so erhitzte Mischung passierte eine wärmeisolierter Haltestrecke (5) mit einer durchschnittlichen Verweilzeit von 12,6 sec. und wird dort in einem Wärmetauscher auf eine Temperatur 60 °C abgekühlt und anschließend auf eine auf 5 °C gekühlte Platte gegeben. Die erhaltene feste Formulierung weist einen Gehalt an Ax-DMDS von 1,42 Gew.-% auf.

## Patentansprüche

1. Feste Formulierung von Astaxanthin-Derivaten in Form einer festen Suspension oder Lösung des Astaxanthin-Derivats in einem für Nahrungsmittel geeigneten Verdünnungsmittel, das einen Schmelzpunkt von wenigstens 40 °C aufweist und das unter für Nahrungsmittel geeigneten Fettsäuren, Fettsäureestern, Fettalkoholen und Wachsen mit einem Schmelzpunkt von wenigstens 40 °C und deren Gemischen ausgewählt ist, wobei das Astaxanthin-Derivat zu wenigstens 70 Gew.-% aus wenigstens einer Verbindung der Formel I 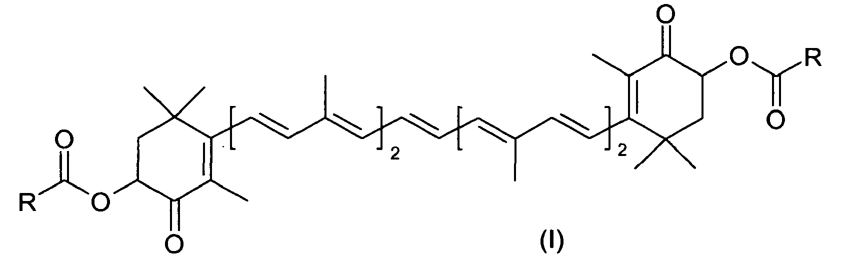 besteht, worin R für einen Rest der Formel 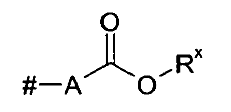 steht, worin # die Anknüpfung an die Carbonylgruppe bedeutet, A für -CH₂CH₂-oder für -CH=CH- steht und R^{x} für C₁-C₄ Alkyl steht.

2. Formulierung nach Anspruch 1, wobei in Formel I der Rest R^{x} Methyl oder Ethyl bedeutet und A für -CH₂CH₂- steht.

3. Formulierung nach einem der vorhergehenden Ansprüche, wobei das Verdünnungsmittel ausgewählt ist unter gesättigten C₁₂-C₃₀-Fettsäuren, Mono-, Di- und Triglyceriden von gesättigten C₁₄-C₂₈-Fettsäuren, Estern gesättigter C₁₄-C₂₈-Fettsäuren mit C₁₀-C₃₀-Fettalkoholen, C₁₆-C₃₀-Fettalkoholen und deren Gemischen.

4. Formulierung nach Anspruch 3, wobei das Verdünnungsmittel ein Hartfett ist.

5. Formulierung nach einem der vorhergehenden Ansprüche, wobei die Formulierung eine schüttfähige partikelförmige Formulierung, insbesondere ein Granulat ist.

6. Formulierung nach Anspruch 5, wobei die Partikel der festen Formulierung einen mittleren Teilchendurchmesser (Gewichtsmittel) im Bereich von 100 µm bis 5 mm aufweisen, wobei wenigstens 90 Gew.-% der Partikel eine Größe im Bereich von 200 µm bis 10 mm aufweisen.

7. Formulierung nach einem der vorhergehenden Ansprüche, wobei die Verbindung der Formel I in der Formulierung zu wenigstens 80 % in der all-trans-Form vorliegt.

8. Formulierung nach einem der vorhergehenden Ansprüche, enthaltend weiterhin wenigstens einen weiteren für Nahrungsmittel geeigneten Zusatz, der unter Oxidationsstabilisatoren, Emulgatoren, Fließhilfsmitteln und Mitteln zur Erhöhung der Viskosität ausgewählt ist.

9. Formulierung nach einem der vorhergehenden Ansprüche mit einem Gehalt an Astaxanthin-Derivat von 1 bis 50 Gew.-%, bezogen auf das Gesamtgewicht der Formulierung.

10. Formulierung nach einem der vorhergehenden Ansprüche in Form einer festen Suspension des Astaxanthin-Derivats in dem für Nahrungsmittel geeigneten Verdünnungsmittel, worin die Partikel des Astaxanthin-Derivats einen volumenmittleren Teilchendurchmesser, bestimmt durch Fraunhoferbeugung, im Bereich von 0,1 bis 50 µm aufweisen.

11. Verfahren zur Herstellung einer Formulierung gemäß einem der vorhergehenden Patentansprüche, umfassend das Vermahlen eines Astaxanthin-Derivats, das zu wenigstens 70 % aus wenigstens einer Verbindung der Formel I besteht, in einer Schmelze des für Nahrungsmittel geeigneten Verdünnungsmittels, gegebenenfalls Erhitzen der beim Vermahlen erhaltenen Suspension, bis das Astaxanthin-Derivat in der Schmelze gelöst ist, und Abkühlen der dabei erhaltenen flüssigen Suspension oder Lösung des Astaxanthin-Derivats in der Schmelze des für Nahrungsmittel geeigneten Verdünnungsmittels.

12. Verfahren nach Anspruch 11, wobei man die flüssige Suspension mittels eines kühlen Gasstroms oder einer kühlen Flüssigkeit, in der das Verdünnungsmittel unlöslich ist, abschreckt.

13. Verwendung einer Formulierung nach einem der Ansprüche 1 bis 10 zur Herstellung von Futtermittelzubereitungen, insbesondere festen Futtermittelzubereitungen, speziell Futtermittelzubereitungen in Form von Pellets.

14. Verwendung einer Formulierung nach einem der Ansprüche 1 bis 10 zur Herstellung von Lösungen des Astaxanthin-Derivats in einem für Nahrungsmittel geeigneten flüssigen Öl.

15. Verfahren zur Herstellung von Futtermittelzubereitungen, insbesondere von festen Futtermittelzubereitungen, speziell von Pellets, umfassend:
a) Bereitstellung einer Formulierung gemäß einem der Ansprüche 1 bis 10;
b) Lösen der Formulierung in einem für Nahrungsmittel geeigneten flüssigen Öl unter Erhalt einer verdünnten flüssigen Lösung des Astaxanthin-Derivats in dem Öl; und
c) Vermischen der in Schritt b) erhaltenen flüssigen Lösung mit den Bestandteilen der Futtermittelzubereitung oder Imprägnieren einer festen Futtermittelzubereitung mit der in Schritt b) erhaltenen flüssigen Lösung.

16. Verfahren nach Anspruch 15, wobei das Lösungsmittel ausgewählt ist unter pflanzlichen und tierischen Ölen, die bei Normaldruck einen Schmelzpunkt unterhalb 40 °C aufweisen.

17. Verfahren nach Anspruch 15 oder 16, wobei das Vermischen bei Temperaturen oberhalb des Schmelzpunktes des flüssigen Verdünnungsmittels erfolgt.

18. Verfahren nach Anspruch 15, 16 oder 17, wobei die in Schritt b) erhaltene verdünnte flüssige Lösung das Astaxanthin-Derivat in einer Konzentration von 10 bis 1000 ppm enthält.

## Claims

1. A solid formulation of astaxanthin derivatives in the form of a solid suspension or solution of the astaxanthin derivative in a diluent suitable for foods which has a melting point of at least 40°C, and which is selected from food-suitable fatty acids, esters of fatty acids, fatty alcohols and waxes having a melting point of at least 40°C and mixtures thereof, where the astaxanthin derivative comprises at least 70% by weight of at least one compound of the formula I 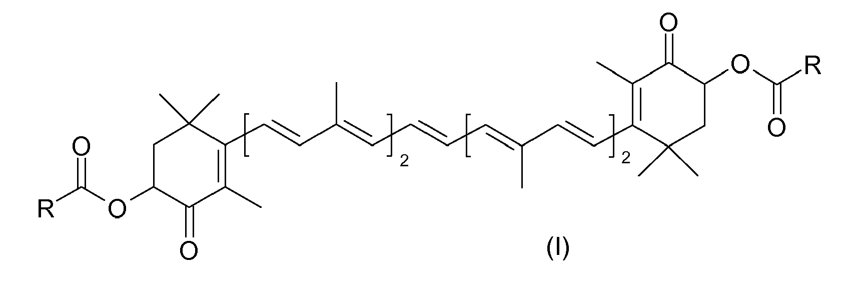 where R is a moiety of the formula 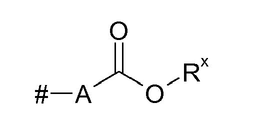 where # means the link to the carbonyl group, A is -CH₂CH₂- or -CH=CH- and R^{x} is C₁-C₄ alkyl.

2. The formulation according to claim 1, wherein in formula I the moiety R^{x} is methyl or ethyl and A is -CH₂CH₂-.

3. The formulation according to any one of the preceding claims, wherein the diluent is selected from saturated C₁₂-C₃₀ fatty acids, mono-, di- and triglycerides of saturated C₁₄-C₂₈ fatty acids, esters of saturated C₁₄-C₂₈ fatty acids with C₁₀-C₃₀ fatty alcohols, C₁₆-C₃₀ fatty alcohols and mixtures thereof.

4. The formulation according to claim 3, wherein the diluent is a hard fat.

5. The formulation according to any one of the preceding claims, wherein the formulation is a pourable particulate formulation, in particular a granular formulation.

6. The formulation according to claim 5, wherein the particles of the solid formulation have a median particle diameter (weight average) in the range from 100 µm to 5 mm, wherein at least 90% by weight of the particles have a size in the range from 200 µm to 10 mm.

7. The formulation according to any one of the preceding claims, wherein at least 80% of the compound of the formula I is present in the formulation in the all-trans form.

8. The formulation according to any one of the preceding claims, further comprising at least one further additive suitable for foods which is selected from oxidation stabilizers, emulsifiers, flow promoters and agents for increasing the viscosity.

9. The formulation according to any one of the preceding claims having a content of astaxanthin derivative of 1 to 50% by weight, based on the total weight of the formulation.

10. The formulation according to any one of the preceding claims in the form of a solid suspension of the astaxanthin derivative in the diluent suitable for foods, in which the particles of the astaxanthin derivative have a volume-average particle diameter determined by Fraunhofer diffraction in the range from 0.1 to 50 µm.

11. A method for producing a formulation according to any one of the preceding claims, which comprises milling an astaxanthin derivative which comprises at least 70% of at least one compound of the formula I in a melt of the diluent suitable for foods, optionally heating the suspension obtained in the milling until the astaxanthin derivative is dissolved in the melt, and cooling the resultant liquid suspension or solution of the astaxanthin derivative in the melt of the diluent which is suitable for foods.

12. The method according to claim 11, wherein the liquid suspension is quenched by means of a cool gas stream or a cool liquid in which the diluent is insoluble.

13. The use of a formulation according to any one of claims 1 to 10 for producing feed preparations, in particular solid feed preparations, especially feed preparations in the form of pellets.

14. The use of a formulation according to any of one of claims 1 to 10 for producing solutions of the astaxanthin derivative in a liquid oil which is suitable for foods.

15. A method for producing feed preparations, in particular solid feed preparations, especially pellets, which comprises:
a) providing a formulation according to any one of claims 1 to 10;
b) dissolving the formulation in a liquid oil which is suitable for foods obtaining a dilute liquid solution of the astaxanthin derivative in the oil; and
c) mixing the liquid solution obtained in step b) with the components of the feed preparation or impregnating a solid feed preparation with the liquid solution obtained in step b).

16. The method according to claim 15, wherein the solvent is selected from vegetable and animal oils which have a melting point below 40°C at atmospheric pressure.

17. The method according to claim 15 or 16, wherein the mixing is performed at temperatures above the melting point of the liquid diluent.

18. The method according to claim 15, 16 or 17, wherein the dilute liquid solution obtained in step b) comprises the astaxanthin derivative at a concentration of 10 to 1000 ppm.

## Revendications

1. Formulation solide de dérivés d'astaxanthine sous forme d'une suspension ou d'une solution solide du dérivé d'astaxanthine dans un diluant approprié pour les aliments, qui présente un point de fusion d'au moins 40°C et qui est choisi parmi les acides gras, les esters d'acide gras, les alcools gras et les cires appropriés pour les aliments, présentant un point de fusion d'au moins 40°C et leurs mélanges, le dérivé d'astaxanthine étant constitué à raison d'au moins 70% en poids par au moins un composé de formule I 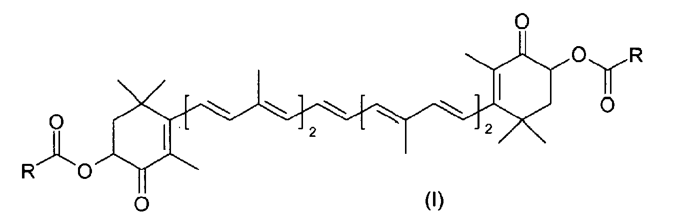 R représentant un radical de formule 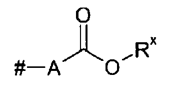 où # signifie la liaison au groupe carbonyle, A représente -CH₂CH₂- ou -CH=CH- et R^{x} représente C₁-C₄-alkyle.

2. Formulation selon la revendication 1, où, dans la formule I, le radical R^{x} signifie méthyle ou éthyle et A représente -CH₂CH₂-.

3. Formulation selon l'une quelconque des revendications précédentes, le diluant étant choisi parmi les acides gras saturés en C₁₂-C₃₀, les monoglycérides, les diglycérides et les triglycérides d'acides gras saturés en C₁₄-C₂₈, les esters d'acides gras saturés en C₁₄-C₂₈ avec des alcools gras en C₁₀-C₃₀, les alcools gras en C₁₆-C₃₀ et leurs mélanges.

4. Formulation selon la revendication 3, le diluant étant une graisse dure.

5. Formulation selon l'une quelconque des revendications précédentes, la formulation étant une formulation sous forme de particules, pouvant être déversée, en particulier un granulat.

6. Formulation selon la revendication 5, les particules de la formulation solide présentant un diamètre moyen de particules (moyenne pondérale) dans la plage de 100 µm à 5 mm, au moins 90% en poids des particules présentant une grosseur dans la plage de 200 µm à 10 mm.

7. Formulation selon l'une quelconque des revendications précédentes, le composé de formule I se trouvant dans la formulation à raison d'au moins 80% dans la configuration all-trans.

8. Formulation selon l'une quelconque des revendications précédentes, contenant en outre au moins un autre additif approprié pour les aliments, qui est choisi parmi les stabilisateurs de l'oxydation, les émulsifiants, les fluidifiants et les agents pour augmenter la viscosité.

9. Formulation selon l'une quelconque des revendications précédentes présentant une teneur en dérivé d' astaxanthine de 1 à 50% en poids, par rapport au poids total de la formulation.

10. Formulation selon l'une quelconque des revendications précédentes sous forme d'une suspension du dérivé d'astaxanthine dans le diluant approprié pour les aliments, les particules du dérivé d'astaxanthine présentant un diamètre moyen volumique des particules, déterminé par diffraction de Fraunhofer, dans la plage de 0,1 à 50 µm.

11. Procédé pour la préparation d'une formulation selon l'une quelconque des revendications précédentes, comprenant le broyage d'un dérivé d'astaxanthine qui est constitué à raison d'au moins 70% par au moins un composé de formule I, dans une masse fondue du diluant approprié pour les aliments, le cas échéant chauffage de la suspension obtenue lors du broyage, jusqu'à ce que le dérivé d'astaxanthine soit dissous dans la masse fondue et refroidissement de la suspension ou de la solution liquide obtenue du dérivé d'astaxanthine dans la masse fondue du diluant approprié pour les aliments.

12. Procédé selon la revendication 11, la suspension liquide étant refroidie brusquement au moyen d'un flux gazeux froid ou un liquide froid dans lequel le diluant est insoluble.

13. Utilisation d'une formulation selon l'une quelconque des revendications 1 à 10 pour la préparation de compositions fourragères, en particulier des compositions fourragères solides, en particulier des compositions fourragères sous forme de pellets.

14. Utilisation d'une formulation selon l'une quelconque des revendications 1 à 10 pour la préparation de solutions du dérivé d'astaxanthine dans une huile liquide appropriée pour les aliments.

15. Procédé pour la préparation de compositions fourragères, en particulier de compositions fourragères solides, en particulier de pellets, comprenant :
a) la mise à disposition d'une formulation selon l'une quelconque des revendications 1 à 10 ;
b) la dissolution de la formulation dans une huile liquide appropriée pour les aliments avec obtention d'une solution liquide diluée du dérivé d'astaxanthine dans l'huile ; et
c) le mélange de la solution liquide obtenue dans l'étape b) avec les constituants de la composition fourragère ou l'imprégnation d'une composition fourragère solide par la solution liquide obtenue dans l'étape b).

16. Procédé selon la revendication 15, le solvant étant choisi parmi les huiles végétales et animales, qui présentent, à pression normale, un point de fusion inférieur à 40°C.

17. Procédé selon la revendication 15 ou 16, le mélange étant réalisé à des températures au-dessus du point de fusion du diluant liquide.

18. Procédé selon la revendication 15, 16 ou 17, la solution liquide diluée obtenue dans l'étape b) contenant le dérivé d'astaxanthine en une concentration de 10 à 1000 ppm.
